# EUROPÄISCHE PATENTSCHRIFT

(11) **EP 2 111 826 B1**
(45) Veröffentlichungstag und Bekanntmachung des Hinweises auf die Patenterteilung: **28.11.2012**
(21) Anmeldenummer: 09156301.5
(22) Anmeldetag: 26.03.2009
(51) Int. Cl.: A61F 2/84

(54) **Einführvorrichtung mit einer Freisetzeinrichtung zur Freisetzung eines von einem Katheter getragenen Gegenstandes sowie Freisetzeinrichtung einer Einführvorrichtung**
Insertion device with a release device for releasing an object held by a catheter and release device of an insertion device
Dispositif d'introduction avec un dispositif de libération pour la libération d'un objet supporté par un cathéter et dispositif de libération d'un dispositif d'introduction

(30) Priorität: 26.04.2008 DE 102008021060
(43) Veröffentlichungstag der Anmeldung: 28.10.2009
(73) Patentinhaber: Biotronik VI Patent AG, 6341 Baar (CH)
(72) Erfinder: Fargahi, Amir, 5242, Birr (CH); Moehl, Raimund, 8127, Forch (CH); Colic, Vesna, 8180, Bülach (CH); Bachmann, Patrice, 8400, Winterthur (CH)
(74) Vertreter: Lindner-Vogt, Karin L.

(56) Entgegenhaltungen:
- EP-A- 1 844 739
- WO-A-2009/091603
- DE-T2- 60 011 355
- US-B1- 6 533 811

## Beschreibung

Die Erfindung betrifft eine Einführvorrichtung mit einer Freisetzeinrichtung zur Freisetzung eines von einem Katheter getragenen Gegenstandes sowie eine Freisetzeinrichtung einer Einführvorrichtung nach den Oberbegriffen der unabhängigen Ansprüche.

Zur Freisetzung von Gegenständen wie z.B. Stents oder bei der Anwendung von Dilatationsballons kommen bekanntermaßen Katheter zum Einsatz. Ein Anwendungsgebiet betrifft z.B. die Angioplastie, bei der Ballonkatheter in Blutgefäße eingeführt, bis zu einer Stenose eingeschoben und dort aufgeweitet werden, um die Verengung des Blutgefäßes zu beseitigen. Üblicherweise ragt am distalen Ende ein Führungsdraht mit kleinem Durchmesser über den Ballonkatheter hinaus.

Bekannt ist aus der DE 600 11 355 T2 eine Einführvorrichtung für eine intravaskuläre Prothese bei der ein Katheter an seinem proximalen Ende in eine Laufvorrichtung eingreift, die eine relative Verschiebung zwischen einer inneren und einer äußeren Hülle des Katheters erleichtert. Zum Freisetzen eines am distalen Ende angeordneten Gegenstands, wie etwa einem Stent, wird die äußere Hülle zum proximalen Ende gezogen. Die Bewegung kann gleichförmig erfolgen, indem die äußere Hülle in die Laufvorrichtung hineingezogen wird.

Auch aus der beispielhaft genannten EP 1 447 058 A1, der US 6 866 669 B2 und der EP 747 021 A2 sind derartige Einführvorrichtungen bekannt.

Bei den vorbekannten Lösungen besteht eine Abhängigkeit zwischen der Gesamtlänge des Katheters und der Stentlänge. Je länger der Stent ist, desto länger ist die Gesamtlänge des Katheters. So ergibt sich beispielsweise eine Gesamtlänge eines Katheters mit einer Stentlänge von 200 mm, die um mindestens 180 mm länger ist als die eines Katheters mit 20 mm Stentlänge. Für einen Anwender, z.B. den Arzt, der den Katheter einführt, bedeutet dies den Einsatz eines längeren Führungsdrahts (Guide Wire) und eine entsprechend komplizierte und unergonomische Handhabung.

In EP 1 844 739 wird eine Freisetzeinrichtung gezeigt, bei der der Außenschaft eine Perforation besitzt. Entlang diese Sollbuchstelle kann der Außenschaft mittels eines Kiels aufgetrennt werden, der Außenschaft wird dann aufgespult.

Der Erfindung liegt die Aufgabe zugrunde, eine verbesserte Einführvorrichtung mit einer Freisetzeinrichtung für einen freizusetzenden Gegenstand sowie eine verbesserte Freisetzeinrichtung einer Einführvorrichtung zu schaffen, die eine leichtere und ergonomische Handhabung ermöglicht.

Die Aufgabe wird erfindungsgemäß durch die Merkmale des unabhängigen Anspruches gelöst. Günstige Ausgestaltungen und Vorteile der Erfindung ergeben sich aus den weiteren Ansprüchen und der Beschreibung.

Die Erfindung geht aus von einer Einführvorrichtung mit einer Freisetzeinrichtung zur Freisetzung eines Gegenstands, insbesondere eines Stützkörpers, der von einem Katheter getragen ist, wobei der Katheter wenigstens einen Außenschaft aufweist, welcher gegen den Gegenstand zu dessen Freisetzung relativ verschieblich ist.

Es wird vorgeschlagen, dass der Katheter an seinem proximalen Ende eine Aufspuleinrichtung aufweist, die zur Erzeugung einer relativen Verschiebung des Außenschafts einen proximalen Abschnitt des Außenschafts aufspult. Das Aufspulen versetzt den Außenschaft in eine Gleitbewegung weg vom distalen Ende des Katheters zum proximalen Ende. Die Gleitbewegung kann kontrolliert und präzise erfolgen. Das Aufspulen ermöglicht eine kompakte Ausführung des Katheters mit einer Länge unabhängig von der Länge des freizusetzenden Gegenstands, der beispielsweise ein Stent sein kann. Ebenso kann die Aufspuleinrichtung den Anwender entlasten und die Vortriebskraft für das Zurückziehen des Außenschafts bei der Freisetzung des Gegenstands wenigstens teilweise übernehmen. Eine Freisetzung kann sehr kontrolliert und exakt erfolgen. Bei einer Motorisierung der Aufspuleinrichtung kann das Verschieben des Außenschafts kontinuierlich und gleichmäßig erfolgen. Ebenso ist denkbar, dass eine variable Verschiebegeschwindigkeit manuell oder automatisiert verwirklicht werden kann, um etwa zu Beginn des Freisetzens den Außenschaft langsam und gegen Ende des Freisetzens den Außenschaft schneller zurückzuziehen.

Bevorzugt kann die Freisetzeinrichtung eine Schneidvorrichtung aufweisen, mit der der zum proximalen Ende des Katheters bewegte Abschnitt des Außenschafts aufschneidbar ist. Vorzugsweise kann die Schneidvorrichtung wenigstens eine Schneide aufweisen, welche den Außenschaft in axialer Richtung aufschneidet. Durch das Aufschneiden des Außenschafts kann das freie Ende des Außenschafts in der Aufspuleinrichtung gefasst und aufgespult werden. Dadurch kann vermieden werden, dass bei Stützkörpern wie etwa Stents mit großen Längenausdehnungen der Katheter länger ausgeführt werden muss als bei kürzeren Stützkörpern, um eine sichere Freisetzung des Stützkörpers zu gewährleisten. Der Katheter kann unabhängig von der Länge des Stützkörpers ausgebildet werden. Bei der Herstellung von Kathetern für unterschiedliche freizusetzende Gegenstände wie etwa Stents können preiswerte Gleichteile verwendet werden.

Vorteilhaft kann die Freisetzeinrichtung ein Griffteil des Katheters bilden. Dies erlaubt eine besonders ergonomische Handhabung des Katheters. Ebenso kann die Freisetzeinrichtung einen T-Körper als Endstück des Katheters bilden.

Die Aufspuleinrichtung kann vorzugsweise eine Rolle umfassen, die einseitig am Katheter angeordnet ist. Es kann ein Schnitt im Außenschaft erzeugt werden und die aufgeschnittene Schaftmantelfläche von der Aufspuleinrichtung aufgespult werden.

Alternativ kann die Aufspuleinrichtung wenigstens zwei am Katheter, vorzugsweise symmetrisch angeordnete Rollen umfassen. Hier können wenigstens zwei Schnitte erzeugt werden und der aufgeschnittene Abschnitt des Außenschafts in getrennte Flächenbereiche geteilt werden, die jeweils separat von der Aufspuleinrichtung gefasst und aufgespult werden können. Dies erleichtert das Aufspulen, das mit weniger Kraftaufwand erfolgen kann.

Weiterhin geht die Erfindung aus von einer Freisetzeinrichtung einer Einführvorrichtung zur Freisetzung eines Gegenstands, insbesondere eines Stützkörpers, der von einem Katheter an dessen distalem Ende getragen ist, wobei der Katheter wenigstens einen Außenschaft aufweist, welcher gegen den Gegenstand zu dessen Freisetzung relativ verschieblich ist.

Es wird eine vorgeschlagen, eine Aufspuleinrichtung vorzusehen, die zur Erzeugung einer relativen Verschiebung des Außenschafts einen proximalen Abschnitt des Außenschafts aufspult. Das Aufspulen versetzt den Außenschaft in eine Gleitbewegung weg vom distalen Ende des Katheters zum proximalen Ende. Die Gleitbewegung kann kontrolliert und präzise erfolgen. Eine Freisetzung des Gegenstands kann sehr kontrolliert und exakt erfolgen, da die kleine Dimension des Handhabungsteils vorteilhaft eine optimale Handhabung erlaubt. Bei einer Motorisierung der Aufspuleinrichtung kann das Verschieben des Außenschafts kontinuierlich und gleichmäßig erfolgen. Ebenso ist denkbar, dass eine variable Verschiebegeschwindigkeit manuell oder automatisiert verwirklicht werden kann, um etwa zu Beginn des Freisetzens zum genauerem Positionieren den Außenschaft langsam und gegen Ende des Freisetzens den Außenschaft schneller zurückzuziehen. Die Aufspuleinrichtung kann günstigerweise an einen üblichen Katheter angeschlossen werden.

Die Aufspuleinrichtung kann an eine Schneidvorrichtung koppelbar sein, mit der der zum proximalen Ende des Katheters bewegte Abschnitt des Außenschafts aufschneidbar ist, wenn sich der Außenschaft zum Freisetzen des Gegenstands am distalen Ende des Katheters zum proximalen Ende bewegt.

Wenigstens eine Rolle kann in der Aufspuleinrichtung vorgesehen sein, auf die der Außenschaft wenigstens bereichsweise aufspulbar ist.

Die wenigstens eine Rolle an einem axialen Ende weist einen kleineren Durchmesser auf als am gegenüberliegenden axialen Ende, es kann der Außenschaft bei konstanter Umdrehungsgeschwindigkeit mit veränderlicher Geschwindigkeit bewegt werden.

Eine verbesserte Handhabung ergibt sich, wenn die wenigstens eine Rolle durch eine Arretierung arretierbar sein kann. Dies ermöglicht einen definierten Beginn der Freisetzung sowie eine gute Kontrolle der Bewegung des Außenschafts, die auch kontrolliert unterbrochen werden kann.

Die Aufspuleinrichtung zum Aufwickeln kann günstigerweise mit einen Federelement so gekoppelt sein, dass durch die Federkraft das Aufspulen und/oder die Bewegung des Außenschafts unterstützt werden kann. Dies ermöglicht eine besonders einfache und kontrollierte Handhabung der Aufspuleinrichtung.

Die Aufspuleinrichtung zum Aufspulen des Außenschafts kann händisch antreibbar sein, z. B. mit einem Kurbelelement.

Die Aufspuleinrichtung kann alternativ oder zusätzlich zum Aufspulen des Außenschafts einen elektrischen Antrieb umfassen. Damit kann das Aufspulen des Außenschafts ohne Krafteinsatz des Anwenders erfolgen.

Die Aufspuleinrichtung kann ein Betätigungselement aufweisen, das in einer ersten Stellung die wenigstens eine Rolle zur Ausführung einer Drehbewegung freigibt und in einer weiteren Stellung die wenigstens eine Rolle blockiert. Vorteilhafterweise kann so eine definierte und gesteuerte Bewegung des Außenschafts erreicht werden.

Die Erfindung ist nachfolgend beispielhaft, anhand von in Zeichnungen dargestellten Ausführungsbeispielen, näher erläutert. Es zeigen in schematischer Darstellung:
- Fig. 1: eine erste bevorzugte Ausgestaltung der Erfindung mit einer Aufspuleinrichtung zum Aufspulen eines aufgeschnittenen Außenschafts zur Freisetzung eines Stents an einem distalen Ende eines Katheters;
- Fig. 2: ein Detail der Ausgestaltung der Fig. 1;
- Fig. 3: eine weitere bevorzugte Ausgestaltung der Erfindung mit einem einseitig aufgeschnittenen Außenschaft;
- Fig. 4a, 4b: Detailansichten zweier miteinander zusammenwirkenden Rollen einer bevorzugten Aufspuleinrichtung;
- Fig. 5: eine Draufsicht auf eine Einführvorrichtung mit einer bevorzugten Aufspuleinrichtung;
- Fig. 6: eine Seitenansicht der bevorzugten Einführvorrichtung in Fig. 5 ohne Gehäuse und Aufspuleinrichtung;
- Fig. 7a, 7b: alternative Ausgestaltungen einer bevorzugten Einführvorrichtung mit unterschiedlich zu einem Katheter orientierten Rollen;
- Fig. 8a, 8b: weitere alternative Ausgestaltungen einer bevorzugten Einführvorrichtung mit unterschiedlich zu einem Katheter orientierten Rollen; und
- Fig. 9: eine bevorzugte Ausgestaltung einer Rolle einer Aufspuleinrichtung;
- Fig. 10a, 10b: einen bevorzugten Auslösemechanismus einer Aufspuleinrichtung mit einer Rolle (Fig. 10a) mit einem Detail des Auslösemechanismus (Fig. 10b);

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Zur Vermeidung unnötiger Wiederholungen wird bei nicht näher beschriebenen Elementen in einer Figur auf die jeweilige Beschreibung der Elemente in vorstehenden Figuren verwiesen.

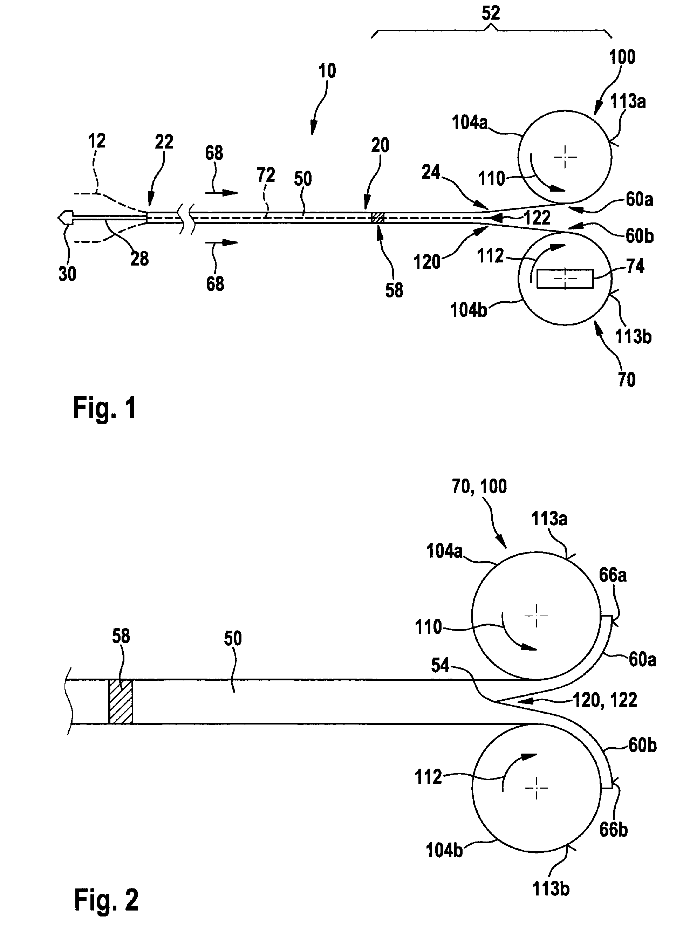

Fig. 1 zeigt eine erste bevorzugte Ausgestaltung mit einer bevorzugten ersten Einführvorrichtung 10 mit einer bevorzugten Freisetzeinrichtung 70 zur Freisetzung eines Gegenstands 12, insbesondere eines Stützkörpers, der an einem distalen Ende 22 eines Katheters 20 getragen ist, wobei der Katheter 20 wenigstens einen Außenschaft 50 aufweist, welcher gegen den Gegenstand 12 zu dessen Freisetzung in einer Ziehrichtung 68 relativ verschieblich ist.

Der Katheter 20 weist an seinem proximalen Ende 24 eine Aufspuleinrichtung 100 auf, die zur Erzeugung der relativen Verschiebung des Außenschafts 50 einen proximalen Abschnitt 52 des Außenschafts 50 aufspult. Der proximale Abschnitt 52 des Außenschafts 50 korrespondiert mit der Länge des Gegenstands 12, der z.B. ein selbstexpandierender Stent sein kann. Proximal und distal beziehen sich auf die Position des Anwenders, das proximale Ende 24 ist benachbart zum Anwender, während das distale Ende 22 entfernt vom Anwender ist.

Der Außenschaft 50 ist um einen Innenschaft 28 angeordnet, wobei am distalen Ende 22 des Katheters 20 eine Spitze 30 zur Führung des Katheters 20 über einem Einführungsdraht (Guide Wire) in an sich üblicher Weise vorgesehen ist, wobei der Gegenstand 12 den Innenschaft 28 umgibt und zum Einführen in z.B. ein Blutgefäß innerhalb des Außenschafts 50 eingepasst ist.

Die Aufspuleinrichtung 100 umfasst in diesem Ausführungsbeispiel zwei symmetrisch zum Katheter 20 bzw. Außenschaft 50 angeordnete Rollen 104a, 104b, die mit einem nicht dargestellten Antrieb antreibbar sind, z.B. einer Kurbel oder einem Elektromotor.

Die Freisetzeinrichtung 70 umfasst neben der Aufspuleinrichtung 100 eine Schneidvorrichtung 120 mit wenigstens einer Schneide 122, mit der der zum proximalen Ende 24 des Katheters 20 bewegter Abschnitt 52 des Außenschafts 50 entlang der Längserstreckung 72 des Außenschafts 50 aufschneidbar ist. Bei Verwendung von zwei Rollen 104a, 104b ist eine symmetrische Trennung des Außenschafts 50 in zwei Teile 60a, 60b zweckmäßig.

Der Außenschaft 50 kann am proximalen Ende des Katheters 20 aufgeschnitten sein und dessen Enden an den Rollen 104a, 104b befestigt werden. Werden die Rollen 104a, 104b gedreht, werden die Teile 60a, 60b auf einer Mantelfläche 113a (Rolle 104a) bzw. 113b (Rolle 104b) der Rollen 104a, 104b abgelegt und mit den Enden 66a, 66b befestigt, wie in dem Detail der Fig. 2 deutlicher zu erkennen ist.

Gleichzeitig führt dies zu einer Verschiebung des Außenschafts 50 in Richtung der Rollen 104a, 104b, wobei die Schneidvorrichtung 120 den Abschnitt 52 weiter auftrennt, bis zum Beispiel ein Stoppelement 58 erreicht ist. Ein Stoppen des Aufschneidens kann auch erreicht werden, indem nur eine begrenzte Anzahl der Umdrehung von Rollen 104a, 104b zugelassen wird.

Die Rollen 104a, 104b werden mit gegensinnigem Drehsinn 110, 112 gedreht, die Rolle 104a z.B. im Gegenuhrzeigersinn und die Rolle 104b im Uhrzeigersinn. Die Drehung kann mit einem Antrieb 74 erfolgen, z.B. einer händisch betätigbaren Kurbel, oder auch elektrisch (nicht dargestellt).

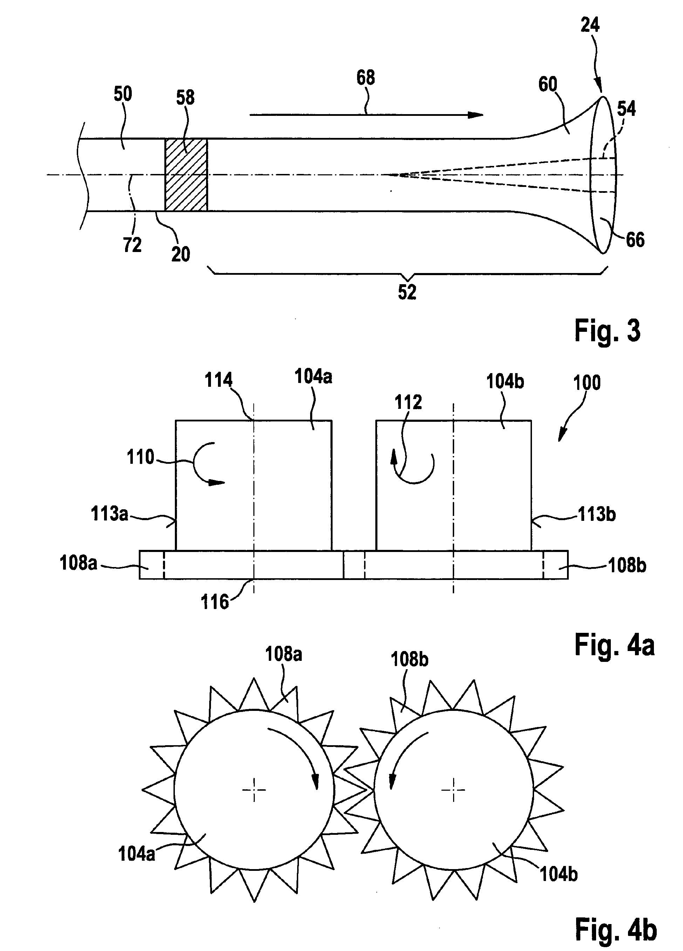

Wird der Antrieb 74 an der Rolle 104b betätigt und diese in Drehung versetzt, kann durch eine günstige Ausgestaltung der Aufspuleinrichtung 100 (Fig. 4a, 4b) die andere Rolle 104a mit bewegt werden. Dazu ist an jeder Rolle 104a, 104b an einem Ende jeweils ein Zahnrad 108a, 108b angeordnet. Die Zahnräder 108a, 108b kämmen miteinander, so dass die Bewegung einer Rolle z.B. 104b die andere Rolle, z.B. 104a, mitnimmt und eine symmetrische Aufspulung ermöglicht (Fig. 4a, 4b).

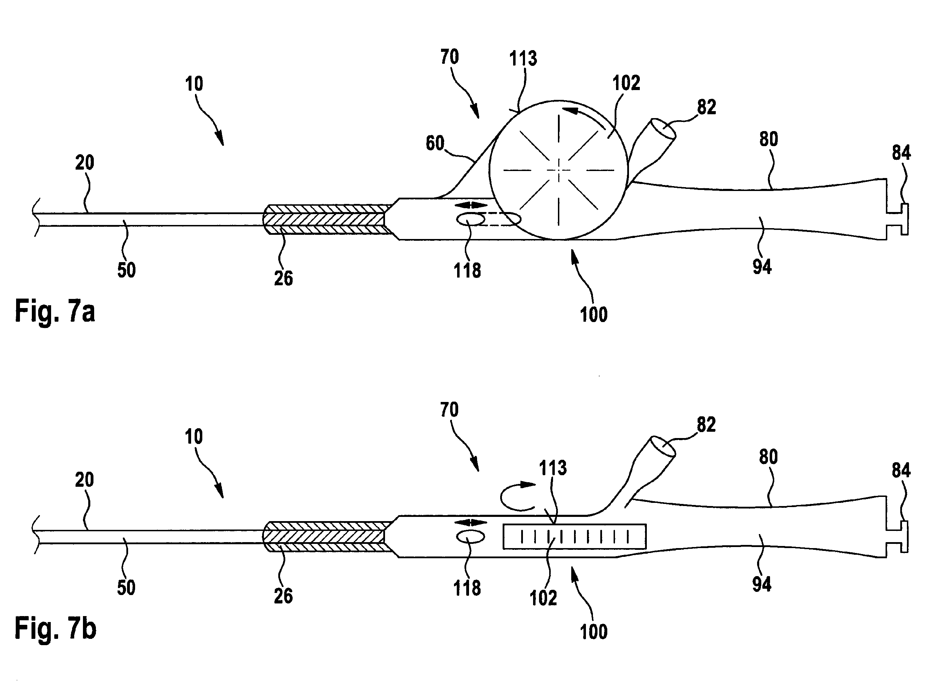

Fig. 3 zeigt eine Variante der Erfindung, bei der in dem Außenschaft 50 nicht mehrere Schnitte angebracht werden, sondern nur ein einseitiger Schnitt 54 von einem proximalen freien Ende 66 des Außenschafts 50 bis zu einem Stoppelement 58 einlang der Längserstreckung 72 des Katheters 20 erzeugt wird. Die Aufspuleinrichtung (nicht dargestellt) weist dann vorzugsweise nur eine einzige Rolle zum Aufspulen des Außenschafts 50 auf, wie in dem Ausführungsbeispiel in Fig. 7a, 7b näher erläutert wird.

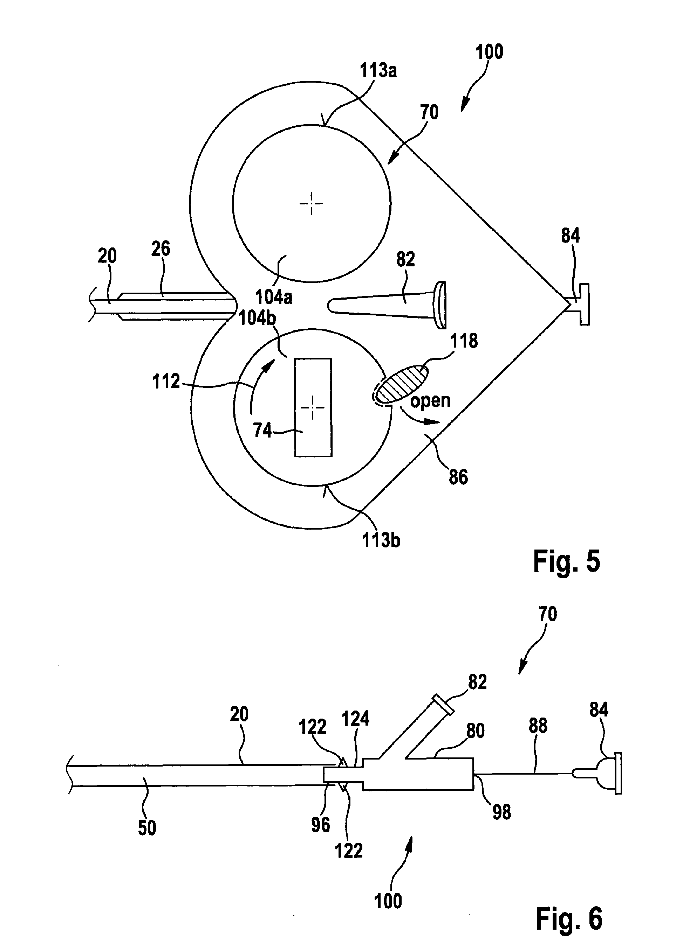

Fig. 5 zeigt eine bevorzugte Freisetzungseinrichtung 70 mit zwei Rollen 104a, 104b in einem Gehäuse 86 in Draufsicht, und Fig. 6 zeigt einen Endkörper 80 des Katheters 20 in Seitenansicht, auf den das Gehäuse 86 mit der Freisetzungseinrichtung 70 angeordnet (Gehäuse und Aufspuleinrichtung in der Seitenansicht in Fig. 6 nicht dargestellt) sein kann. Der Endkörper 80 kann auch in das Gehäuse 86 der Freisetzeinrichtung 70 integriert sein

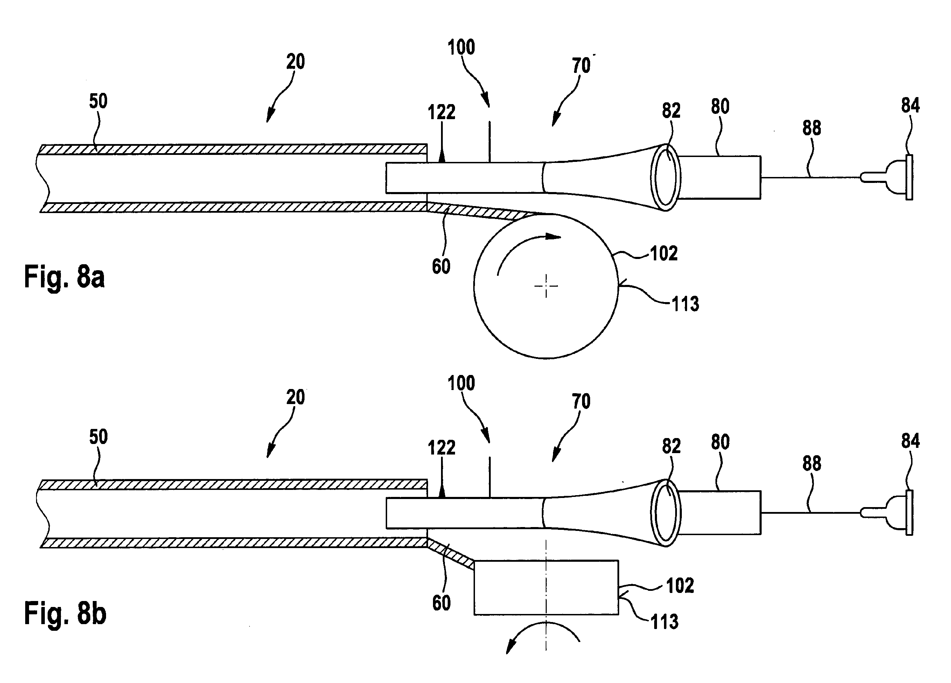

Die Rollen 104a, 104b liegen symmetrisch zueinander und drehen sich bei Betätigung gegensinnig. Die Rollen 104a, 104b können mit einer an einer der Rollen 104b angeordneten Arretierung 118 fixiert oder freigegeben werden. Der Innenschaft 28 des Katheters 20 (Fig. 1) kann z.B. mittels eines Metallschafts 88 (Fig. 6; Fig. 8a, 8b) im Gehäuse 86 fixiert werden. Zur Stabilisierung ist angrenzend an das Gehäuse 86 ein Knickschutz 26 am Katheter 20 angebracht, der den Katheter 20 ummantelt.

Ein Anschluss 82, z.B. in Gestalt eines so genannten Luer Locks, dient zum üblichen Spülen eines Raums zwischen Innenschaft 28 und Außenschaft 50, und ein am freien Ende angeordneter Anschluss 84, der ebenso als Luer Lock ausgebildet sein kann, dient zum Einführen eines Führungsdrahts (Guide Wire) sowie zum Spülen dieses Bereichs. Distal zum Anschluss 82 sind zwei symmetrisch angeordnete Schneiden 122 an einem Ansatz 124 des Endkörpers 80 angeordnet, die den Außenschaft 50 beim Zurückziehen von Innen heraus zerschneiden. Denkbar wäre auch eine Anordnung, dass der Außenschaft 50 mit außen liegenden Schneiden (nicht dargestellt) längs zerteilt wird. Distal und proximal sind am Endkörper 80 jeweils Dichtungen 96 und 98 angeordnet, um den Raum zwischen Innenschaft 28 und Außenschaft 50 bzw. Metallschaft 88 und Endkörper 80 bzw. Außenschaft 50 abzudichten.

Der bereits teilweise geschlitzte Außenschaft 50 wird zum Aufspulen auf die Mantelflächen 113a, 113b der Rollen 104a, 104b an diesen befestigt, wie in den Fig. 1 und 2 angedeutet wurde. Durch Drehen der Rollen 104a, 104b wird der Außenschaft 50 aufgespult und damit automatisch zum proximalen Ende 24 des Katheters 20 zurückgezogen. Somit wird der Gegenstand 12 (Fig. 1), etwa ein selbstexpandierender Stent, freigesetzt.

Fig. 7a, 7b illustrieren schematisch weitere bevorzugte Ausführungsbeispiele einer erfindungsgemäßen Einführvorrichtung 10 mit einer Freisetzeinrichtung 70, die eine Aufspuleinrichtung 100 umfasst, die nur eine Rolle 102 aufweist und eine einseitige Schneidevorrichtung umfasst, wie sie in Fig. 3 schematisch dargestellt ist. Die Rolle 102 ist einseitig an dem Katheter 20 angeordnet. Die Freisetzeinrichtung 70 ist in einen Endkörper 80 des Katheters 20 integriert und bildet einen Griffteil des Katheters 20 mit einem Griffbereich 94, den der Anwender in der Hand halten kann. Die Rolle 102 kann entweder im Uhrzeigersinn oder im Gegenuhrzeigersinn gedreht werden, wobei für den Anwender nur die Drehung in einer Richtung gestattet und die Gegenrichtung gesperrt ist.

Der Katheter 20 ist außenumfänglich mit einem Knickschutz 26 versehen, ragt in den Endkörper 80 hinein und kann ebenso wie vorstehend beschrieben mit einem Metallschaft am Endkörper 80 befestigt sein.

Fig. 7a zeigt eine Ausgestaltung, bei der die Rolle 102 der Aufspuleinrichtung 100 hochkant angeordnet ist, so dass die Rolle 102 mit ihrer Kreisfläche parallel neben dem Katheter 20 steht. In Fig. 7b ist eine alternative Ausgestaltung abgebildet, bei der der Katheter 20 tangential zur Rolle 102 angeordnet ist. Die vorhandene Schneidvorrichtung ist nicht explizit dargestellt.

Mit einer z.B. als hin und her beweglichem Schieber ausgebildeten Arretierung 118 kann die Rolle 102 fixiert oder freigegeben werden, was durch einen Doppelpfeil angedeutet ist. Die Rolle 102 ist distal zum Griffbereich 94 des Endkörpers 80 angeordnet. In beiden Ausgestaltungen kann der Anwender, der das Endteil am Griffbereich 94 hält, die Rolle 102 z.B. mit dem Daumen drehen, so dass eine einhändige Bedienung der Freisetzeinrichtung 70 bzw. der Aufspuleinrichtung 100 der Einführvorrichtung 10 möglich ist. Ein Stoppen des Aufschneidens kann mit einem Stoppelement 58 (Fig. 1, 2, 3) erreicht werden, oder indem nur eine begrenzte Anzahl der Umdrehung von Rolle 102 zugelassen wird.

Die Fig. 8a und 8b zeigen eine weitere bevorzugte Ausgestaltung der Erfindung mit einem als T-Körper ausgebildeten Endkörper 80, bei dem die Rolle 102 der Aufspuleinrichtung 100 tangential (Fig. 8a) oder parallel neben dem Katheter 20 bzw. dem Endkörper 80 (Fig. 8b) angeordnet ist.

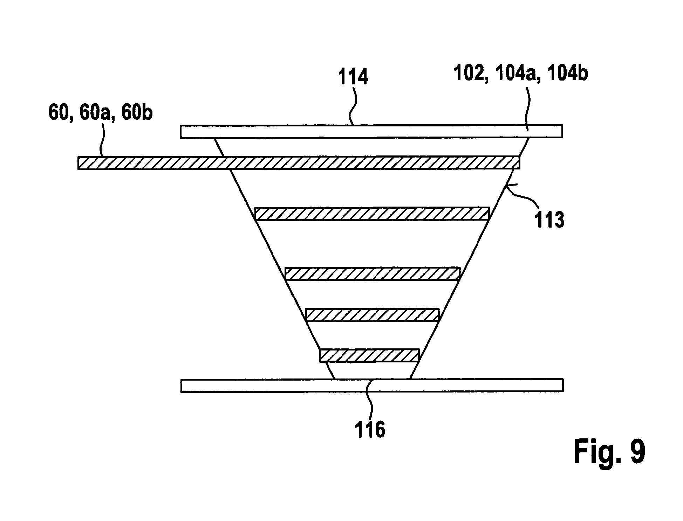

Die Rollen 102, 104a, 104b in den vorstehend beschriebenen Ausführungsbeispielen der Aufspuleinrichtung 100 können eine zylinderförmige Mantelfläche 113 aufweisen oder auch eine konisch zulaufende Form aufweisen, wie in Fig. 9 dargestellt ist. An einem axialen Ende 114 weist die Rolle 102, 104a, 104b einen größeren Durchmesser der Mantelfläche 113 auf als an ihrem gegenüberliegenden axialen Ende 116. Dies führt dazu, dass die Teile 60, 60a, 60b des Außenschafts 50 bei konstanter Umdrehungsgeschwindigkeit der Rolle 102, 104a, 104b unterschiedlich schnell aufgespult werden, je nachdem, auf welcher axialen Höhe die Teile 60, 60a, 60b abgelegt werden. Zu Beginn der Freisetzung kann z.B. auf das dünnere Ende der Rolle 102, 104a, 104b gespult werden, so dass der Außenschaft 50 mit geringer Geschwindigkeit zurückgezogen wird und gegen Ende der Freisetzung auf das dickere Ende der Rolle 102, 104a, 104b gespult werden, was eine schnellere Bewegung des Außenschafts 50 verursacht.

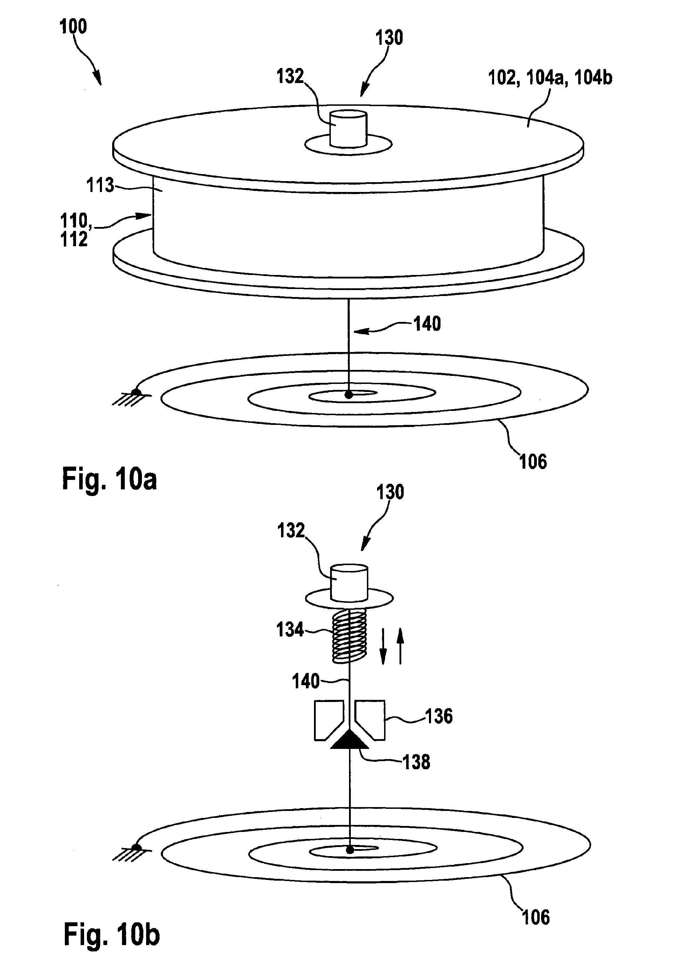

Die Figuren 10a und 10b zeigen ein Detail einer vorteilhaften Aufspuleinrichtung 100, bei dem die Rolle 102, 104a, 104b mit einem Auslösemechanismus 130 mechanisch oder elektrisch gedreht werden kann. Der Auslösemechanismus erstreckt sich entlang einer zentralen Achse 140 der Rolle 102, 104a, 104b. Ein Betätigungselement 132 kann gegen eine Federkraft eines z.B. als Axialfeder ausgebildeten Federelements 134 nach unten bewegt werden. Durch die Bewegung wird ein Gegenelement 138 aus einer Bremse 136 bewegt und die Achse 140 damit freigegeben. Die Bremse 136 kann z.B. Bremsbacken umfassen, in die das Gegenelement 138 eingreift, wenn die Rolle 102, 104a, 104b blockiert ist. Die Achse 140 ist mit einem als Drehfeder ausgebildeten Federelement 106 gekoppelt, welches die Achse 140 und damit die Rolle 102, 104a, 104b in eine Drehbewegung versetzen kann, wenn das Gegenelement 138 von der Bremse 136 entkoppelt ist. Statt des Federelements 106 kann auch ein elektrischer Antrieb vorgesehen sein. Die Spule 102, 104a, 104b kann per Knopfdruck bedient werden und bietet die Möglichkeit, unterschiedliche Aufspulgeschwindigkeiten zu realisieren. Ein Stoppen des Aufschneidens kann z.B. erreicht werden, indem nur eine begrenzte Anzahl der Umdrehung der Rollen 102, 104a, 104b und/oder eine limitierte Bewegung des Federelements 106 zugelassen wird.

Insgesamt erlaubt das Aufschneiden und das Aufspulen des Außenschafts 50 die Bereitstellung eines kurzen Handhabungsteils des Katheters 20. Dies führt vorteilhaft zu einer einfacheren Handhabung und einem verkürzten Verschiebewegs des Katheters 20 über den Führungsdraht (Guide Wire). Zusätzlich erlaubt die Vereinfachung des Freisetzprozesses, insbesondere über eine einfache Knopfbedienung der Freisetzeinrichtung, eine genauere und homogenere Positionierung des Gegenstands, z.B. eine Stents im Blutgefäß.

## Patentansprüche

1. Freisetzeinrichtung einer Einführvorrichtung (10) zur Freisetzung eines Gegenstands (12), insbesondere eines Stützkörpers, , der von einem Katheter (20) an dessen distalem Ende (22) getragen ist, wobei der Katheter (20) wenigstens einen Außenschaft (50) aufweist, welcher gegen den Gegenstand (12) zu dessen Freisetzung relativ verschieblich ist, und wobei die Freisetzeinrichtung eine Aufspuleinrichtung (100) aufweist, die zur Erzeugung einer relativen Verschiebung des Außenschafts (50) einen proximalen Abschnitt (52) des Außenschafts (50) aufspult, und wobei in der Aufspuleinrichtung (100) wenigstens eine Rolle (102, 104) vorgesehen ist, auf die der Außenschaft (50) wenigstens bereichsweise aufspulbar ist, **dadurch gekennzeichnet, dass** die wenigstens eine Rolle (102, 104) an einem axialen Ende (114) einen kleineren Durchmesser aufweist als am gegenüberliegenden axialen Ende (116).

2. Freisetzeinrichtung nach Anspruch 1, **dadurch gekennzeichnet, dass** mit der Aufspuleinrichtung (100) eine Schneidvorrichtung (120) koppelbar ist, mit der der zum proximalen Ende (24) des Katheters (20) bewegte Abschnitt (52) des Außenschafts (50) aufschneidbar ist, wenn sich der Außenschaft (50) zum Freisetzen des Gegenstands (12) am distalen Ende (22) des Katheters (20) zum proximalen Ende (24) bewegt.

3. Freisetzeinrichtung nach einem der Ansprüche 1 oder 2, **dadurch gekennzeichnet, dass** die wenigstens eine Rolle (102, 104) durch eine Arretierung (118) arretierbar ist.

4. Freisetzeinrichtung nach einem der Ansprüche 1 bis 3, **dadurch gekennzeichnet, dass** die Aufspuleinrichtung (100) zum Aufwickeln mit einem Federelement (106) so gekoppelt sind, dass die Federkraft das Aufspulen und/oder die Bewegung des Außenschafts (50) unterstützt.

5. Freisetzeinrichtung nach einem der Ansprüche 1 bis 4, **dadurch gekennzeichnet, dass** die Aufspuleinrichtung (100) zum Aufspulen des Außenschafts (50) händisch antreibbar ist.

6. Freisetzeinrichtung nach einem der Ansprüche 1 bis 5, **dadurch gekennzeichnet, dass** die Aufspuleinrichtung (100) zum Aufspulen des Außenschafts (50) einen elektrischen Antrieb umfasst.

7. Freisetzeinrichtung nach einem der Ansprüche 1 bis 6, **dadurch gekennzeichnet, dass** die Aufspuleinrichtung (100) ein Betätigungselement (132) aufweist, das in einer ersten Stellung die wenigstens eine Rolle (102, 104) zur Ausführung einer Drehbewegung freigibt und in einer weiteren Stellung die wenigstens eine Rolle (102, 104) blockiert.

## Claims

1. A release device of an insertion apparatus (10) for releasing an object (12), in particular a supporting body, which is carried by a catheter (20) at the distal end (22) thereof, wherein the catheter (20) has at least one outer shaft (50), which is relatively displaceable against the object (12) for release thereof, and wherein the release device has a winding device (100), which winds up a proximal portion (52) of the outer shaft (50) to generate a relative displacement of the outer shaft (50), and wherein at least one roller (102, 104), onto which the outer shaft (50) can be wound, at least over regions, is provided in the winding device (100), **characterised in that** the at least one roller (102, 104) has a smaller diameter at one axial end (114) than at the opposite axial end (116).

2. The release device according to Claim 1, **characterised in that** the winding device (100) can be coupled to a cutting device (120), with which the portion (52) of the outer shaft (50) moved toward the proximal end (24) of the catheter (20) can be cut when the outer shaft (50) moves toward the proximal end (24) to release the object (12) at the distal end (22) of the catheter (20).

3. The release device according to one of Claims 1 or 2, **characterised in that** the at least one roller (102, 104) can be locked by a locking mechanism (118).

4. The release device according to one of Claims 1 to 3, **characterised in that**, for winding, the winding device (100) is coupled to a spring element (106) such that the resilience assists the winding and/or the movement of the outer shaft (50).

5. The release device according to one of Claims 1 to 4, **characterised in that** the winding device (100) can be driven manually to wind up the outer shaft (50).

6. The release device according to one of Claims 1 to 5, **characterised in that** the winding device (100) comprises an electric drive to wind up the outer shaft (50).

7. The release device according to one of Claims 1 to 6, **characterised in that** the winding device (100) has an actuation element (132), which, in a first position, releases the at least one roller (102, 104) to carry out a rotational movement and, in a further position, blocks the at least one roller (102, 104).

## Revendications

1. Dispositif de libération d'un appareil d'introduction (10) pour la libération d'un objet (12), en particulier d'un élément porteur, qui est porté par un cathéter (20) à l'extrémité distale (22) de celui-ci, le cathéter (20) ayant au moins une tige externe (50), qui peut être déplacée de manière relative contre l'objet (12) pour la libération de celui-ci, et le dispositif de libération ayant un dispositif d'enroulement (100), qui enroule une partie proximale (52) de la tige externe (50) pour générer un déplacement relatif de la tige externe (50), et au moins un rouleau (102, 104), sur lequel la tige externe (50) peut être enroulée, au moins sur des régions, étant disposé dans le dispositif d'enroulement (100), **caractérisé par le fait que** le ou les rouleaux (102, 104) ont un diamètre plus petit à une extrémité axiale (114) qu'à l'extrémité axiale opposée (116).

2. Dispositif de libération selon la revendication 1, **caractérisé par le fait que** le dispositif d'enroulement (100) peut être couplé à un dispositif de coupe (120), au moyen duquel la partie (52) de la tige externe (50) déplacée en direction de l'extrémité proximale (24) du cathéter (20) peut être coupée lorsque la tige externe (50) se déplace en direction de l'extrémité proximale (24) pour libérer l'objet (12) à l'extrémité distale (22) du cathéter (20).

3. Dispositif de libération selon l'une des revendications 1 ou 2, **caractérisé par le fait que** le ou les rouleaux (102, 104) peuvent être verrouillés par un mécanisme de verrouillage (118).

4. Dispositif de libération selon l'une des revendications 1 à 3, **caractérisé par le fait que**, pour un enroulement, le dispositif d'enroulement (100) est couplé à un élément de ressort (106) de telle sorte que l'élasticité aide l'enroulement et/ou le déplacement de la tige externe (50).

5. Dispositif de libération selon l'une des revendications 1 à 4, **caractérisé par le fait que** le dispositif d'enroulement (100) peut être entraîné manuellement pour enrouler la tige externe (50).

6. Dispositif de libération selon l'une des revendications 1 à 5, **caractérisé par le fait que** le dispositif d'enroulement (100) comprend un entraînement électrique pour enrouler la tige externe (50).

7. Dispositif de libération selon l'une des revendications 1 à 6, **caractérisé par le fait que** le dispositif d'enroulement (100) a un élément d'actionnement (132), qui, dans une première position, libère le ou les rouleaux (102, 104) pour réaliser un mouvement de rotation et, dans une autre position, bloque le ou les rouleaux (102, 104).
